# FASCICULE DE BREVET EUROPEEN

(11) **EP 0 661 955 B1**
(45) Date de publication et mention de la délivrance du brevet: **23.04.1997**
(21) Numéro de dépôt: 93920881.5
(22) Date de dépôt: 14.09.1993
(51) Int. Cl.: A61F 2/02

(54) **KIT A USAGE MEDICAL COMPOSE D'UN FILTRE ET DE SON DISPOSITIF DE MISE EN PLACE DANS LE VAISSEAU**
MEDIZINISCHE EINRICHTUNG MIT EINEM FILTER UND EINER VORRICHTUNG ZUM EINSETZEN IN DAS GEFÄSS
MEDICAL KIT COMPRISING A FILTER AND A DEVICE FOR PLACING SAID FILTER IN THE VESSEL

(30) Priorité: 28.09.1992 FR 9211915
(43) Date de publication de la demande: 12.07.1995
(73) Titulaire: BENTEX TRADING S.A., L-2311 Luxembourg (LU)
(72) Inventeur: LEFEBVRE, Jean-Marie, F-59800 Lille (FR)
(74) Mandataire: Bruin, Cornelis Willem
(86) Numéro de dépôt international: FR9300884
(87) Numéro de publication internationale: WO9407431

(56) Documents cités:
- EP-A- 0 408 245
- FR-A- 2 567 405
- FR-A- 2 580 504
- US-A- 3 952 747

## Description

La présente invention concerne la filtration des caillots circulant dans le flux sanguin. Elle concerne plus particulièrement un ensemble constitué par un filtre et son dispositif de mise en place dans le vaisseau.

Pour empêcher la migration de caillots provenant des veines inférieures du corps dans le coeur et l'artère pulmonaire et donc pour éviter l'embolie, il est connu d'implanter des dispositifs filtrants, dénommés ci-après filtres, devant permettre de faire obstacle au passage des caillots tout en autorisant l'écoulement du flux sanguin à travers le vaisseau.

Il existe un grand nombre de types de filtres. On connaît en particulier le filtre dénommé GREENFIELD qui est constitué de fils préformés en zigzags, réunis par un capuchon. Ces fils sont disposés pour former un cône, dont le sommet est le capuchon, lesdits zigzags formant l'enveloppe du cône. Les extrémités des fils sont pourvues de crochets d'amarrage, permettant d'assurer la fixation du filtre sur la paroi interne du vaisseau.

La mise en place d'un tel filtre est réalisée à partir d'une gaine d'introduction à l'intérieur de laquelle est inséré le filtre, les fils élastiques étant maintenus sensiblement selon l'axe longitudinal de la gaine. Après avoir positionné l'extrémité distale de la gaine d'introduction à l'endroit où doit être placé le filtre dans le vaisseau, le largage de celui-ci est réalisé à l'aide d'un élément poussoir qui est une tige actionnable depuis l'extérieur de la gaine d'introduction et qui, prenant appui sur le filtre, permet de déplacer celui-ci tout en maintenant fixe la gaine d'introduction Une fois largués dans le vaisseau, les fils élastiques se déploient en cône à l'intérieur du vaisseau, les crochets d'extrémité des fils venant se fixer sur la paroi interne du vaisseau.

Depuis ce filtre GREENFIELD, on a proposé d'autres filtres reposant sur le même principe de fonctionnement. Il s'agit en particulier du filtre décrit dans le document FR 2 570 288. Le filtre décrit dans ce document présente une forme générale conique avec plusieurs pattes réunies vers leurs têtes formant pointe ogivale et allant en divergeant vers leur autre extrémité. Il s agit d'un filtre monobloc qui est formé dans une feuille mince d'un matériau à élasticité appropriée. Les pattes en question sont donc des lames plates de faible épaisseur. Dans le document précité, FR 2 570 288, lesdites pattes sont effilées à leur extrémité libre. Pour sa mise en place à l'intérieur du vaisseau, ce filtre est placé dans un tube d'introduction. On introduit d'abord un guide métallique souple à l'intérieur de la veine, puis, en utilisant ce guide métallique, on introduit ensuite un premier tube introducteur relativement mince, que l'on utilise comme tube de guidage pour le passage du tube d'introduction qui contient le filtre.

Ainsi en plus du guide métallique souple, la mise en place d'un tel filtre nécessite l'utilisation de deux tubes introducteurs.

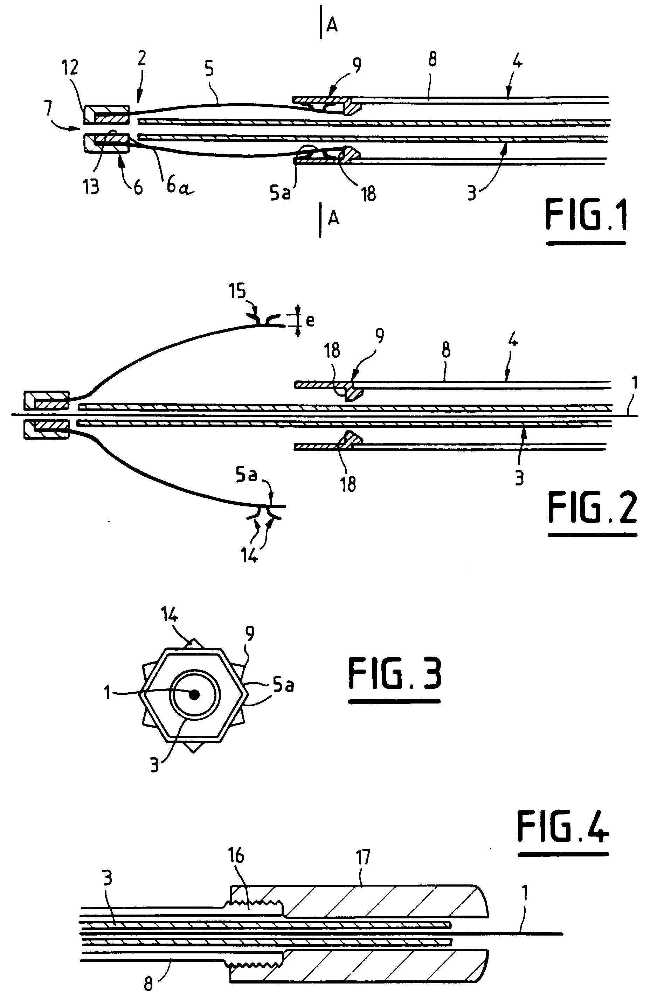

Le document US-A-3 952 747 (D1), qui peut être considéré comme l'état de la technique le plus pertinant pour la revendication 1 de la demande, décrit (voir figures 4 et 5) un kit à usage médical, composé d'un filtre (10), d'un poussoir (32) et d'un tue introducteur (40), dans lequel
le filtre (10) est formé par des pattes élastiques (16) déployées sensiblement suivant une corolle conique issue d'une tête ogivale (14) et
le tube introducteur (40) est muni à son extrémité distale d'une capsule cylindrique rigide (42) de diamètre intérieur D4, ladite capsule (42) comportant un décrochement interne annulaire vers son extrémité la plus proximale (c.a.d. au voisinage de l'épaulement taraudé 24c), apte à servir de butée pour les extrémités (16a) des pattes (16) du filtre (10), les dimensions respectives des différents éléments précités étant telles qu'en position d'introduction du filtre à l'intérieur du vaisseau, le sommet (14) du filtre étant tourné vers l'avant, les extrémités (16a) des pattes (16) sont repliées à l'intérieur de la capsule (42), l'extrémité distale du poussoir (32) étant en appui contre la partie interne dudit sommet (14).

D'autres filtres connus sont décrits dans les documents FR-A-2 580 504 et FR-A-2 567 405.

Le demandeur a constaté différents inconvénients aux dispositifs actuellement utilisés. D'une part, le premier tube introducteur à travers lequel doit transiter le second tube introducteur contenant le filtre, doit être d'un diamètre somme toute relativement important puisque, comme cela est indiqué dans le document FR 2 570 288, le second tube introducteur peut présenter un diamètre intérieur de 3,6 mm. Ainsi le premier tube introducteur peut avoir un diamètre extérieur qui peut être de l'ordre de 5 mm. Ces tubes sont dans un matériau plastique souple, qui doit cependant être suffisamment rigide pour pouvoir progresser à l'intérieur du vaisseau tout en délimitant un espace intérieur apte à recevoir le second tube introducteur. Il arrive, en fonction des sinuosités des vaisseaux, en particulier s'agissant de la veine fémorale gauche, que le premier tube introducteur fasse l'objet de pliures rédhibitoires empêchant le transit normal du second tube introducteur.

Le but que s'est fixé le demandeur est de proposer un kit composé d'un filtre et de son dispositif de mise en place qui pallie les inconvénients constatés en ce qu'il est de conception simple et qu'il ne nécessite pas de tubes introducteurs de gros diamètre.

Ce but est parfaitement atteint par le kit de l'invention qui est composé d'un filtre, d'un guide métallique souple, d'un poussoir et d'un tube introducteur. Le filtre est formé par des pattes élastiques déployées sensiblement suivant une corolle conique issue d'une tête ogivale, il est percé à son sommet d'un petit orifice de diamètre D1; le guide métallique a un diamètre extérieur D2; le poussoir est un tube creux dont l'évidement intérieur a un diamètre D3; le tube introducteur, en matière plastique, est muni à son extrémité distale d'une capsule cylindrique rigide de diamètre intérieur D4. La capsule comporte un décrochement interne, annulaire, vers son extrémité la plus proximale, apte à servir de butée pour les extrémités des pattes du filtre. Ce décrochement annulaire assure également le centrage du poussoir. Les dimensions respectives des différents éléments précités sont telles que D4 est supérieur à D3 > D1 > D2 de telle sorte que, en position d'introduction du filtre à l'intérieur du vaisseau, le sommet du filtre est tourné vers l'avant, les extrémités de ses pattes étant repliée à l'intérieur de la capsule, l'extrémité distale du poussoir étant en appui contre la partie interne dudit sommet.

La mise en place du filtre grâce au kit de l'invention, est réalisée en introduisant d'abord le guide métallique souple à l'intérieur du vaisseau, puis en enfilant sur celui-ci le filtre lui-même qui est positionné en bout du tube introducteur, les pattes étant repliées et placées à l'intérieur de la capsule terminant ledit tube introducteur. Ainsi on comprend que lors du transit à l'intérieur du vaisseau de l'ensemble constitué par le filtre, le tube introducteur et le poussoir, le filtre lui-même n'est pas enfermé dans un tube introducteur mais est extérieur à celui-ci. Seules les extrémités libres des pattes du filtre sont bloquées dans la capsule entre la face intérieure de ladite capsule et la surface extérieure du poussoir.

On comprend, dans ces conditions, que le tube introducteur peut avoir un diamètre plus faible par exemple de l'ordre de 3 mm.

C'est le maintien du guide métallique souple à l'intérieur du vaisseau pendant l'introduction des autres éléments du kit qui permet d'éviter tout risque de blocage du filtre dans les sinuosités du vaisseau. On peut également remarquer que le filtre, tenu uniquement par l'extrémité libre de ses pattes, présente une certaine flexibilité facilitant le passage de cet ensemble dans les parties de vaisseau particulièrement sinueuses. Bien sûr il importe que le maintien desdites pattes entre la capsule et le poussoir soit suffisant. Pour ce faire, la capsule a une longueur qui est d'au moins 10 mm, pour une longueur de patte comprise entre 40 et 60 mm.

De préférence, le filtre comporte six pattes dont la largeur, au niveau de l'extrémité proximale, est sensiblement égale ou inférieure à la moitié du diamètre intérieur D4 de la capsule. Ainsi en position repliée, les six pattes sont jointives et forment un hexagone régulier sensiblement inscrit dans la capsule.

Dans le cas où les extrémités des pattes comportent des moyens d'accrochage avec la paroi interne du vaisseau, de préférence ces moyens consistent en deux crochets de sens antagonistes, tels que ceux décrits dans le document FR 2 660 189. Plus précisément, chaque crochet est obtenu par emboutissage d'une portion triangulaire de la lame formant l'extrémité distale de la patte; de plus, par rapport au plan de ladite patte, il présente une configuration en forme de coude. Cette disposition particulière diminue la hauteur dudit crochet au-dessus de la patte et réduit en conséquence l'espacement nécessaire entre le poussoir et la capsule pour le logement de chaque patte lors du positionnement du filtre. Cette configuration ne diminue cependant en rien le bon arrimage du filtre dans la paroi du vaisseau.

Afin d'éviter toute fausse manoeuvre lors de la mise en place du filtre, le tube introducteur est terminé à son extrémité distale par un épaulement, qui est destiné à rester à l'extérieur du corps du patient; de plus le kit comporte un capuchon creux de protection qui est solidarisable, par exemple par vissage, audit épaulement et qui recouvre l'extrémité proximale du poussoir, qui dépasse du tube introducteur lorsque ledit capuchon est en position de solidarisation. Ainsi l'opérateur ne peut pas involontairement déplacer le poussoir; il faut pour le faire qu'il désolidarise le capuchon de protection et le retire pour avoir accès à l'extrémité proximale du poussoir.

L'invention sera mieux comprise à la lecture de la description qui va être faite d'un exemple de réalisation du kit de filtration à usage médical, composé d'un guide métallique souple, d'un filtre, d'un poussoir et d'un tube introducteur, illustré par le dessin annexé dans lequel :
La figure 1 est une vue schématique en coupe du filtre prépositionné dans la capsule terminant l'extrémité distale du tube introducteur,
La figure 2 est une vue schématique en coupe du kit de filtration, après largage du filtre à l'intérieur de la veine, et
La figure 3 est une vue en coupe transversale de la capsule du filtre, du poussoir et du guide selon la ligne A,A de la figure 1.
La figure 4 est une vue schématique en coupe de l'extrémité proximale du kit.

Le kit de filtration à usage médical selon l'invention est composé de quatre éléments, à savoir un guide métallique souple 1, un filtre 2, un poussoir 3 et un tube introducteur 4.

Le guide métallique est bien connu par ailleurs, étant déjà d'un usage fréquent. Il s'agit d'un fil métallique souple. Il a un diamètre extérieur donné D1.

Le filtre 2 est formé de pattes élastiques 5 qui sont fixées à la tête 6 du filtre. Cette tête 6 est percée d'un orifice de passage 7 de diamètre intérieur D2, supérieur au diamètre D1 précité.

Le poussoir 3 est un tube souple, creux, dont le diamètre de l'évidement intérieur est légèrement supérieur à D1. Le diamètre extérieur du poussoir est référencé D3.

Le tube introducteur 4 est une gaine plastique et souple 8, terminée par une capsule rigide 9. Il s'agit par exemple d'un petit tube cylindrique en métal sur lequel est montée à chaud l'extrémité de la gaine 8. La capsule 9 a un diamètre intérieur D4 qui est sensiblement supérieur au diamètre extérieur D3 du poussoir 3. Elle comporte vers son extrémité la plus proximale, c'est-à-dire vers le tube introducteur 4 un décrochement annulaire 18.

On a représenté sur la figure 1 le filtre 2 prépositionné sur le tube introducteur 8. Dans cette configuration, la tête 6 du filtre est tournée vers l'avant, c'est-à-dire vers ce qui sera l'extrémité distale après introduction dans la veine. Les pattes 5 sont ramenées longitudinalement le long de l'axe du filtre et introduites à l'intérieur de la capsule 9. Elles viennent sensiblement en butée contre le décrochement interne annulaire 18, au fond de la capsule 9. Quant au poussoir 3, il est placé à l'intérieur du tube introducteur 4, passe dans l'évidement intérieur du décrochement annulaire 18 qui favorise son centrage dans la capsule 9 et dépasse de l'extrémité distale de celui-ci jusqu'à être en contact avec la partie interne 6a de la tête 6.

Sur la figure 4 on a représenté l'extrémité distale du kit, destinée à rester à l'extérieur du corps du patient. Le tube introducteur 4 est terminé par un épaulement 16 taraudé, sur lequel est solidarisé par vissage un capuchon de protection 17. Ce capuchon 17 est creux et s'étend au delà de l'extrémité distale du poussoir 3.

Pour la mise en place du filtre à l'intérieur de la veine 10, on introduit d'abord le guide métallique 1 jusqu'à l'emplacement où doit être positionné le filtre 2.

Ceci étant fait, on enfile sur l'extrémité proximale du guide, qui se trouve donc à l'extérieur du corps du patient, le filtre 2, le poussoir 3 et le tube introducteur 4 dans l'état où ils sont représentés aux figures 1 et 4. Cet enfilage se fait en faisant pénétrer ladite extrémité proximale du guide 1 successivement à travers l'orifice 7 de la tête 6 du filtre 2, et l'évidement intérieur du poussoir 3.

Cet ensemble constitué du filtre 2, du poussoir 3 et du tube introducteur 4, est poussé dans la veine 10 jusqu'à l'emplacement où le filtre 2 doit être posé.

Le largage du filtre 2 est obtenu en maintenant fixe le tube introducteur 4 et en déplaçant vers l'avant le poussoir 3. Ceci ne peut être fait qu'après avoir dévissé le capuchon de protection 17 rendant ainsi accessible à l'opérateur l'extrémité distale du poussoir 3. Ce déplacement repousse la tête 6 du filtre 2 et corrélativement l'extrémité 5a des pattes 5 à l'intérieur de la capsule 9 jusqu'à ce que lesdites extrémités 5a sortent de celle-ci.

Les pattes 5, ainsi libérées de leur emprisonnement dans la capsule 9, se déploient en corolle du fait de leur élasticité naturelle et le filtre 2 forme un cône, les extrémités 5a des pattes 5 prenant appui sur la paroi interne 11 de la veine 10.

Dans un exemple préféré de réalisation, le filtre 2 est formé de six pattes 5 élastiques. Il s'agit de lames métalliques préformées et traitées thermiquement pour avoir en position normale la forme courbe en S telle qu'elle apparaît à la figure 2. La tête 6 du filtre est constituée de deux pièces 12, 13 : une première pièce ogivale creuse et une seconde pièce cylindrique 13. Les pattes 5, disposées symétriquement et accolées les unes aux autres sur la périphérie intérieure de la première pièce ogivale 12 sont bloquées en position par l'introduction de la seconde pièce cylindrique 13.

Le filtre 2 possède également des crochets d'amarrage 14 qui sont situés sur les extrémités libres 5a des pattes 5. De préférence, il y a, sur chaque patte 5, deux crochets 14 et 14' inclinés de manière antagoniste, comme illustré sur la figure 2. Cette forme de crochet est connue par le document FR 2 660 189.

Avantageusement chaque crochet est obtenu par découpe et pliage d'une forme en V réalisée dans la zone centrale de l'extrémité distale 5a de chaque patte 5.

Sur la représentation de la figure 3, il s'agit d'un filtre comportant six pattes 5 dont la largeur l est sensiblement inférieure à la moitié du diamètre intérieur D4 de la capsule 9. Ainsi, une fois positionnées à l'intérieur de la capsule 9, les extrémités 5a de ces six pattes 5 adoptent une position en forme d'hexagone régulier.

Avantageusement les crochets 14 présentent un coude 15, réduisant leur hauteur e par rapport au plan de la patte 5a. Il est préférable que cette hauteur e corresponde sensiblement à la hauteur de l'arc de l'hexagone régulier inscrit dans le cercle figurant la face intérieure de la capsule 9.

Le choix de ces caractéristiques permet d'assurer un logement optimal de l'extrémité 5a des pattes 5 à l'intérieur de la capsule 9, et autour du poussoir 3 de manière à obtenir un largage homogène du filtre 2 à l'intérieur de la veine, sous l'action du poussoir 3.

L'invention n'est pas limitée au mode de réalisation qui vient d'être décrit à titre d'exemple non exhaustif. En particulier, on pourrait utiliser d'autres types de filtre que celui précisément décrit, en particulier celui du document FR 2 570 288, pour autant que l'orifice du sommet ait un diamètre supérieur à celui du guide métallique afin de permettre le passage de celui-ci mais inférieur au diamètre extérieur du poussoir afin que celui-ci puisse prendre appui sur la face intérieure dudit sommet.

## Revendications

1. Kit à usage médical, composé d'un filtre (2), d'un guide métallique souple (1), d'un poussoir (3)et d'un tube introducteur (4), dans lequel
a) le filtre qui est formé par des pattes élastiques (5) déployées sensiblement suivant une corolle conique issue d'une tête ogivale (6), est percé à son sommet d'un petit orifice (7)de diamètre D1,
b) le guide métallique (1) a un diamètre extérieur D2,
c)le poussoir (3) est un tube creux dont l'évidement intérieur a un diamètre D3,
d) le tube introducteur (4) est muni à son extrémité distale d'une capsule cylindrique rigide (9) de diamètre intérieur D4, ladite capsule (9) comportant un décrochement interne annulaire (18) vers son extrémité la plus proximale, apte à servir de butée pour les extrémités (5a) des pattes (5) du filtre,
les dimensions respectives des différents éléments précités étant telles que D4 > D3 > D1 > D2 de telle sorte que, en position d'introduction du filtre (2) à l'intérieur du vaisseau (10), le sommet du filtre étant tourné vers l'avant, les extrémités (5a) de ses pattes (5) sont repliées à l'intérieur de la capsule (9), l'extrémité distale du poussoir étant en appui contre la partie interne (6a) dudit sommet (6).

2. Kit à usage médical selon la revendication 1 caractérisé en ce que la capsule (9) a une longueur d'au moins 10 mm, pour une longueur de patte (5) commprise entre 40 et 60 mm.

3. Kit à usage médical selon l'une des revendications 1 à 2 caractérisé en ce que le filtre (2) comporte six pattes (5) dont la largeur, au niveau de l'extrémité proximale (5a), est sensiblement égale ou inférieure à la moitié du diamètre intérieur D4 de la capsule (9).

4. Kit à usage médical selon l'une des revendications 1 à 3 caractérisé en ce que chaque extrémité (5a) des pattes (5) comporte deux crochets (14) de sens antagonistes.

5. Kit à usage médical selon la revendication 4 caractérisé en ce que chaque crochet (14) présente une configuration en forme de coude.

6. Kit à usage médical selon la revendication 1 caractérisé en ce que le tube introducteur (4) à son extrémité proximale est terminé par un épaulement (16) et en ce que le kit comporte également un capuchon creux de protection (17) solidarisable, par exemple par vissage, audit épaulement et recouvrant l'extrémité proximale du poussoir lorsqu'elle lui est solidarisée.

## Claims

1. Kit for medical use, consisting of a filter (2), a flexible metal guide (1), a plunger (3) and an introducer (4), in which
a) the filter which is formed by elastic legs (5) deployed substantially in a conical corolla emanating from an ogival head (6) has a small orifice (7) of diameter D1 passing through its tip,
b) the metal guide (1) has an external diameter D2,
c) the plunger (3) is a hollow tube, the internal cavity of which has a diameter D3,
d) the introduction tube (4) is provided at its distal end with a rigid cylindrical capsule (9) of internal diameter D4, the said capsule (9) having an internal annu lar step (18) towards its most proximal end, suitable to act as a stop for the ends (5a) of the legs (5) of the filter,
the respective dimensions of the different above-mentioned elements being such that D4 > D3 > D1 > D2 so that, in the position for introduction of the filter (2) into the vessel (10), the tip of the filter being directed forwards, the ends (5a) of its legs (5) are folded back within the capsule (9), the distal end of the plunger resting against the internal part (6a) of the said tip (6).

2. Kit for medical use as described in claim 1, characterised by the fact that the capsule (9) has a length of at least 10 mm, for a leg (5) length of between 40 and 60 mm.

3. Kit for medical use as described in either of claims 1 or 2, characterised by the fact that the filter (2) has six legs (5) of which the width, at the proximal end (5a), is substantially equal to or less than half of the internal diameter D4 of the capsule (9).

4. Kit for medical use as described in one of claims 1 to 3, characterised by the fact that each of the ends (5a) of the legs (5) has two hooks (14) facing in opposite direction.

5. Kit for medical use as described in claim 4, characterised by the fact that each hook (14) is elbow-shaped.

6. Kit for medical use as described in claim 1, characterised by the fact that the introduction tube (4) is terminated at its proximal end by a shoulder (16) and by the fact that the kit also includes a hollow protective cap (17) attachable to the sad shoulder, for example by screwing, and covering the proximal end of the plunger when it is attached to it.

## Patentansprüche

1. Medizinischer Bausatz mit einem Filter (2), einer flexiblen Führung (1) aus Metall, einem Ausstoßer (3) und einem Einführungsrohr (4), bei dem
a) der Filter, der aus elastischen Lappen (5) gebildet ist, die sich im wesentlichen entsprechend einer Blütenkrone ausgehend von einem spitzbogenförmigen Kopf (6) entfalten, an seiner Spitze von einer kleinen Öffnung (7) mit dem Durchmesser D1 durchbohrt ist;
b) die metallische Führung (1) einen Außendurchmesser D2 aufweist;
c) der Ausstoßer (3) ein hohles Rohr ist, dessen innere Aussparung einen Durchmesser D3 aufweist;
d) das Einführungsrohr (4) an seinem fernen Ende mit einer zylindrischen, starren Kapsel (9) mit dem Innendurchmesser D4 versehen ist, welche Kapsel (9) an ihrem am nächsten liegenden Ende einen internen, ringförmigen Vorsprung (18) aufweist, der dafür geeignet ist, als Anschlag für die Enden (5a) der Lappen (5) des Filters zu dienen,
wobei die jeweiligen Abmessungen der oben genannten unterschiedlichen Elemente derart sind, daß D4 > D3 > D1 > D2 ist, so daß in derjenigen Stellung, in der der Filter (2) in das Innere des Gefäßes (10) eingefügt ist, und bei nach vorne gerichteter Spitze des Filters die Enden (5a) der Lappen (5) in das Innere der Kapsel (9) zurückgezogen sind, wobei das ferne Ende des Ausstoßers sich gegen den internen Teil (6a) der Spitze (6) abstützt.

2. Medizinischer Bausatz nach Anspruch 1, dadurch **gekennzeichnet**, daß die Kapsel (9) eine Länge von wenigstens 10 mm für eine Gesamtlänge des Lappens (5) zwischen 40 und 60 mm aufweist.

3. Medizinischer Bausatz nach einem der Ansprüche 1 der 2, dadurch **gekennzeichnet**, daß der Filter (2) sechs Lappen (5) umfaßt, deren Breite auf Höhe des nahen Endes (5a) im wesentlichen gleich oder kleiner als die Hälfte des Innendurchmessers D4 der Kapsel (9) ist.

4. Medizinischer Bausatz nach einem der Ansprüche 1 bis 3, dadurch **gekennzeichnet**, daß jedes Ende (5a) der Lappen (5) zwei gegensätzlich gerichtete Haken (14) umfaßt.

5. Medizinischer Bausatz nach Anspruch 4, dadurch **gekennzeichnet**, daß jeder Haken (14) eine knieförmige Konfiguration aufweist.

6. Medizinischer Bausatz nach Anspruch 1, dadurch **gekennzeichnet**, daß das Einführungsrohr (4) an seinem nahen Ende durch einen Bund (16) abgeschlossen wird, und daß der Bausatz zudem einen hohle Schutzkappe (17) aufweist, die mit dem Bund, beispielsweise durch Verschraubung, verbindbar ist und das nahe Ende des Ausstoßers völlig bedeckt, wenn sie mit dem Bund verbunden ist.
